# EUROPEAN PATENT SPECIFICATION

(11) **EP 0 401 883 B1**
(45) Date of publication and mention of the grant of the patent: **31.08.1994**
(21) Application number: 90200055.3
(22) Date of filing: 09.01.1990
(51) Int. Cl.: A61F 13/04

(54) **Thermoplastic immobilization element for ortopedics**
Thermoplastisches Immobilisationselement für Orthopädie
Elément orthopédique thermoplastique d'immobilisation

(30) Priority: 05.06.1989 BE 8900605
(43) Date of publication of application: 12.12.1990
(73) Proprietor: ORFIT INDUSTRIES, N.V., 2110 WIJNEGEM (BE)
(72) Inventor: van Malderen, Frans, B-2120 Schoten (BE); Cuypers, Steven, B-2230 Schilde (BE)
(74) Representative: Vanderperre, Robert

(56) References cited:
- EP-A- 0 006 607
- DE-A- 2 906 228
- US-A- 3 326 211
- US-A- 4 169 469
- US-A- 4 600 618

## Description

The present invention relates to a thermoplastic immobilization element for orthopedics and this for application on both the human and the animal body.

From the Belgian patent no. 894.522 of the Applicant a thermoplastic extrudable molding material is known that is principally formed through the combination of a so-called HCP (Highly Crystalline Polyester) with a high molecular weight (higher than 5,000); a completely saturated polymeric elastomer and a silica viscosity modifier.

This molding material has notable advantages among others because it can be manipulated with bare hands at 55 degrees Centigrade; it can take any shape; with solidification it tightens around any body; it remains soft and manipulable relatively long; it can be repeatedly re-heated in order to effect modifications; it can be treated mechanically in any manner; etc.

Such material, as described in detail in the aforementioned patent can be applied in a very suitable manner among others for splints for replacing plaster in orthopedics and ergotherapy.

Likewise an immobilization element for orthopedics is already known whereby a transformable material is embedded in a fabric that is provided with a zipper.

With this known immobilization element a two component plastic is taken which must be mixed to create a chemical reaction in order to obtain a hardening of the mixture.

The aforementioned mixture must, obviously before it has hardened, be molded into a doubled walled fabric with zipper and be rolled out in this double walled fabric. Because this entails a manual operation it is clear that it is difficult to obtain a totally even thickness and surface.

The element thus obtained must, already before the aforementioned mixture has hardened, be installed around the body part in question and be fixed around this latter by closing the aforementioned zipper.

A first disadvantage of this known element is that the preparation of it is relatively extensive and requires much time just at the moment that a splint or similar must be installed.

Another disadvantage of the aforementioned known element is that after hardening it is very stiff and offers no more elasticity, in other words the known element can, once it is formed, not be further formed, in other words also not be reused.

Yet another disadvantage of the known element is that it cannot be perforated so that it lets through almost no air.

A thermoplastic immobilization element is also known from US 3.326.211. Said element has to be wrapped around the member to be immobilized in an overlapping manner. It does not comprise means which can provide in an adequate fixation between the overlapping portions.

A similar element is known from US 4.169.469. As disclosed in this document, the element may be provided of fasteners. Although fasteners may be provided, said element cannot provide in adequate support as it comprises a plastic sheet which cannot completely encircle the injured member.

The object of the invention is a thermoplastic immobilization element that excludes the aforementioned and other disadvantages by comprising a cast of moulding material surrounded by a supple material provided with a part of fastener means along each of two opposite edges thereof, said parts being designed to cooperate with each other such that said opposite edges are positioned next to each other, and wherein the cast is comprised of a thermoplastic material that is substantially rigid at ambient temperature and pliable at a moderate higher temperature such that the immobilization element is easily adaptable around a body member while retaining a limited elasticity allowing the immobilization element to be easily removed.

A first advantage is that the immobilization element can be prepared beforehand for a possible use so that it only has to be heated when using it which is very simple and can be effected very quickly.

Another advantage of the immobilization element according to the invention is that it always has and retains a uniform thickness through which no formation of folds can develop.

Yet another advantage of the element according to the invention is that it always retains a limited elasticity so that it can easily be removed.

Another advantage yet of the immobilization element according to the invention is that it can be reused or heated up over and over again, for example to eliminate faults or to alter the shape if such is necessary.

Another advantage is to be seen in the fact that the immobilization element can be supplied in all sorts of perforated versions.

Finally an immobilization element is obtained in this manner that, in relation to immobilization elements of plaster or of the aforementioned two component plastic, can be applied with little technical knowledge and shows the therapeutic advantage that it is removable, all the more because it always retains a certain elasticity, while such immobilization element, in relation to an aforementioned thermoplastic molding material, has the advantage that it shows a pre-cut form and that the fastener, for example a zipper, is already provided so that the actual immobilization can occur in one step, since no additional fastener must be installed, which is certainly the case with the aforementioned thermoplastic molding material.

Embodiments of the thermoplastic immobilisation element according to the invention are defined in the subclaims.

In order to show better the characteristics according to the present invention, a preferred embodiment is described hereafter, as example and without any restrictive character, with reference to the enclosed drawings, in which:
figure 1 shows a top view of an immobilization element according to the invention;
figure 2 shows a cross-section according to line II-II in figure 1;
figure 3 shows an application of the element according to figure 1..

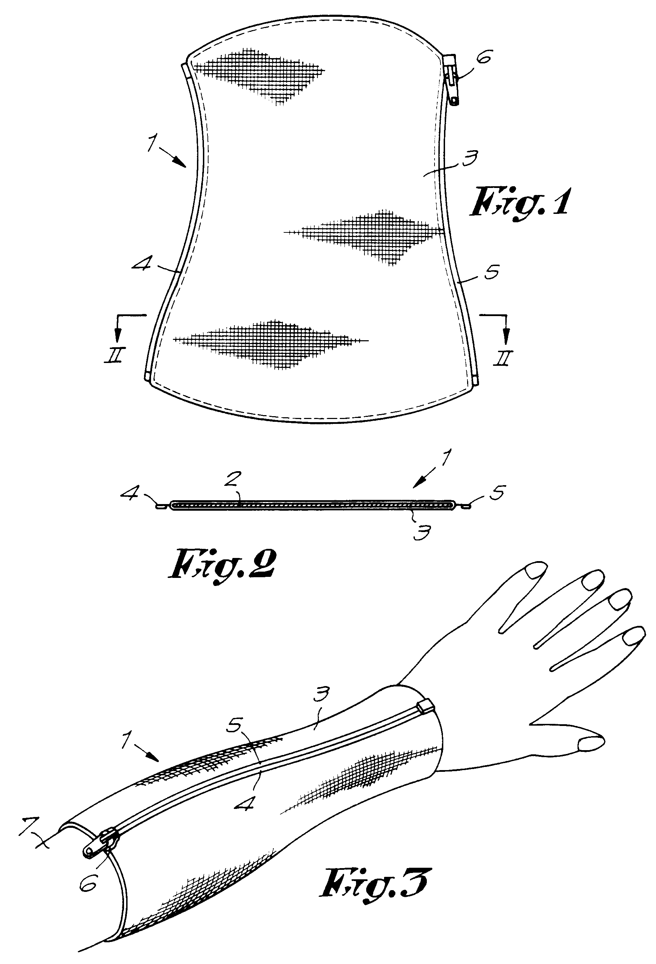

In the figures 1 and 2 an immobilization element 1 according to the invention is shown that is principally meant to splint a forearm.

Such element principally consists of a thermoplastic extrudable molding material 2, for example of the type as described in the Belgian patent no. 894.522, that is installed in a space which for example is formed by a double walled supple material such as a fabric 3, whereby this fabric shows a part of a zipper 4, 5 in this embodiment on two edges lying opposite each other which can be fitted to each other in the known manner and mesh together through the movement of a runner 6 in the linear direction of it.

It is sufficient to heat such an element to a temperature of approximately 55 degrees so that it becomes easily transformable and can be installed around a body part, in this case a forearm 7, after which through the simple closing of the zipper 4, 5 the element 1 is installed and fixed in relation to the body part in question, all of which such that after hardening, through cooling off of the element 1, a suitable splint is obtained.

As already previously described such a splint can be reshaped under influence of heat and if desired be reused after removal.

It is clear that not only a zipper 4, 5 but any other fastener, such as for example a Velcro fastener could be utilized as fastening means.

It is likewise clear that such an immobilization element can and will be produced in all kinds of forms depending on the body part that must be splinted and the dimensions of the body part in question.

The invention is thus in no way restricted to the embodiment described as example and shown in the enclosed drawings.

## Claims

1. Thermoplastic immobilization element (1) for orthopedics comprising a cast of moulding material (2) surrounded by a supple material (3) provided with a part (4,5) of fastener means along each of two opposite edges thereof, said parts (4, 5) being designed to cooperate with each other such that said opposite edges are positioned next to each other, characterized in that the cast (2) is comprised of a thermoplastic material that is substantially rigid at ambient temperature and pliable at a moderate higher temperature such that the immobilisation element is easily adaptable around a body member while retaining a limited elasticity allowing the immobilization element to be easily removed.

2. Immobilization element according to claim 1, characterized in that the thermoplastic material (2) is formed through the combination of a so-called HCP (Highly Crystalline Polyester) with a high molecular weight (higher than 5,000); a completely saturated polymeric elastomer and a silica viscosity modifier.

3. Immobilization element according to claim 1 or 2, characterized in that the supple material (3) is formed by a fabric.

4. Immobilization element according to one of the preceding claims, characterized in that the fastener (4, 5) is formed by a zipper.

5. Immobilization element according to one of the preceding claims 1 through 3, characterized in that the fastener (4, 5) is formed by a Velcro fastener.

## Patentansprüche

1. Thermoplastisches Immobilisierungselement (1) für orthopädische Zwecke, mit einer Gußmasse aus Formmaterial (2), das von einem biegsamen Material (3) umgeben ist, welches entlang zweier einander gegenüberliegender Ränder mit jeweils einem Teil (4, 5) einer Verschlußeinrichtung versehen ist, wobei die Teile (4, 5) derart zusammenwirken, daß die einander gegenüberliegenden Ränder nebeneinanderliegend angeordnet sind, dadurch gekennzeichnet, daß die Gußmasse (2) aus einem thermoplastischen Material besteht, das bei Raumtemperatur im wesentlichen starr und bei einer mäßig höheren Temperatur derart formbar ist, daß das Immobilisierungselement leicht an ein Körperteil anpaßbar ist und gleichzeitig eine begrenzte Elastizität beibehält, die ein leichtes Abnehmen des Immobilisierungselements ermöglicht.

2. Immobilisierungselement nach Anspruch 1, dadurch gekennzeichnet, daß das thermoplastische Material (2) durch Kombinieren eines sogenannten HCP (Highly Crystalline Polyester) mit einem hohen Molekulargewicht (höher als 5000), eines vollständig gesättigten polymeren Elastomers und eines Siliziumdioxid-Viskositätsverbesserers gebildet ist.

3. Immobilisierungselement nach Anspruch 1 oder 2, dadurch gekennzeichnet, daß das biegsame Material (3) ein Gewebe ist.

4. Immobilisierungselement nach einem der vorhergehenden Ansprüche, dadurch gekennzeichnet, daß die Verschlußeinrichtung (4, 5) ein Reißverschluß ist.

5. Immobilisierungselement nach einem der vorhergehenden Ansprüche 1 bis 3, dadurch gekennzeichnet, daß die Verschlußeinrichtung (4, 5) ein Klettverschluß ist.

## Revendications

1. Elément orthopédique thermoplastique d'immobilisation (1) comprenant une pièce moulée en matière de moulage (2) entourée par une matière souple (3) pourvue d'une partie (4,5) de moyens de fixation le long de chacun de deux côtés opposés de celui-ci, lesdites parties (4,5) étant conçues pour coopérer l'une avec l'autre de façon que lesdits côtés opposés soient positionnés l'un à côté de l'autre, caractérisé en ce que la pièce moulée (2) comprend une matière thermoplastique qui est sensiblement rigide à température ambiante et pliable à une température modérément supérieure de façon que l'élément d'immobilisation soit facilement adaptable autour d'un membre du corps tout en conservant une élasticité limitée permettant à l'élément d'immobilisation d'être enlevé facilement.

2. Elément d'immobilisation suivant la revendication 1, caractérisé en ce que la matière thermoplastique (2) est formée par la combinaison de ce que l'on appelle du HCP (polyester hautement cristallin) possédant un poids moléculaire élevé (supérieur à 5.000), un élastomère polymérique complètement saturé et un modificateur de viscosité de la silice.

3. Elément d'immobilisation suivant la revendication 1 ou 2, caractérisé en ce que la matière souple (3) est constituée d'un tissu.

4. Elément d'immobilisation suivant l'une quelconque des revendications précédentes, caractérisé en ce que les moyens de fixation (4,5) sont formés par une fermeture éclair.

5. Elément d'immobilisation suivant l'une des revendications précédentes, caractérisé en ce que les moyens de fixations (4,5) sont constitués d'une fermeture velcro.
